# EUROPEAN PATENT APPLICATION

(11) **EP 4 706 589 A1**
(43) Date of publication of application: **11.03.2026**
(21) Application number: 25199283.0
(22) Date of filing: 01.09.2025
(51) Int. Cl.: A61C 9/00

(54) **ADVANCED INTRAORAL PHOTOGRAMMETRY DEVICE FOR FULL ARCH, COLOR MAP, AND BITE REGISTRATION**

(30) Priority: 04.09.2024 GB 202412959
(71) Applicant: Nulty, Adam Brian, Ixworth, Bury St. Edmunds IP31 2HT (GB)
(72) Inventor: Nulty, Adam Brian, Ixworth, Bury St. Edmunds IP31 2HT (GB)
(74) Representative: Doherty, William

(57) **Abstract**

An advanced intraoral photogrammetry device designed to capture full arch scans, color maps, and bite registration in a single, streamlined procedure. The device features multiple bands of micro cameras oriented at various angles, allowing for comprehensive data capture with minimal actions. The system instantly processes and delivers 3D models and color maps to 3D CAD software, distinguishing it from existing technologies by offering a faster, more accurate, and user-friendly solution.

## Description

### Field of Invention and Background:

The invention relates to a high-precision intraoral photogrammetry device designed for capturing full-arch 3D models, color maps, and bite registration in a single, efficient procedure. Existing intraoral scanning and photogrammetry systems typically require multiple steps, different devices, and are prone to challenges such as obstructed views and incomplete data capture, particularly in complex anatomical regions of the oral cavity. This device is specifically engineered to address these challenges, offering a streamlined and highly accurate alternative.

### Summary of the Invention:

This advanced intraoral photogrammetry device is designed as a single, stick-like apparatus equipped with multiple bands of micro cameras positioned at various angles. It captures photo images and 3D video simultaneously, allowing for instant full-arch scanning, detailed color mapping, and precise bite registration. The device operates with a series of single-click actions to capture comprehensive oral data, which is instantly processed and delivered to 3D CAD software. The system is designed to overcome limitations of current technologies, offering a faster, more accurate, and user-friendly solution.

### Drawings and Figures:

Figure 1: Overview of the intraoral photogrammetry device showing the sensor layout and stick-like design.
Figure 2: Cross-sectional view illustrating the position and orientation of the sensors in the bands around the stick.
Figure 3: Diagram of the single-click full arch scanning process for the upper and lower arches.
Figure 4: Illustration of the bite registration process using the device held against the inner cheek.

### Detailed Description of the Drawings:

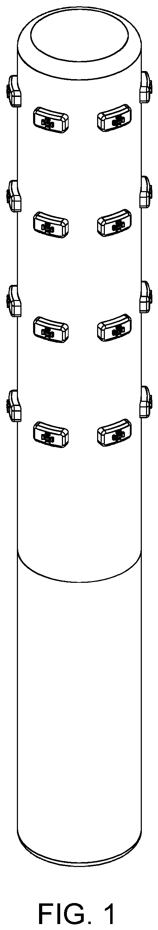

Figure 1: Depicts the overall design of the device, highlighting the sensor placement and the ergonomic stick shape for easy insertion and maneuvering within the mouth.

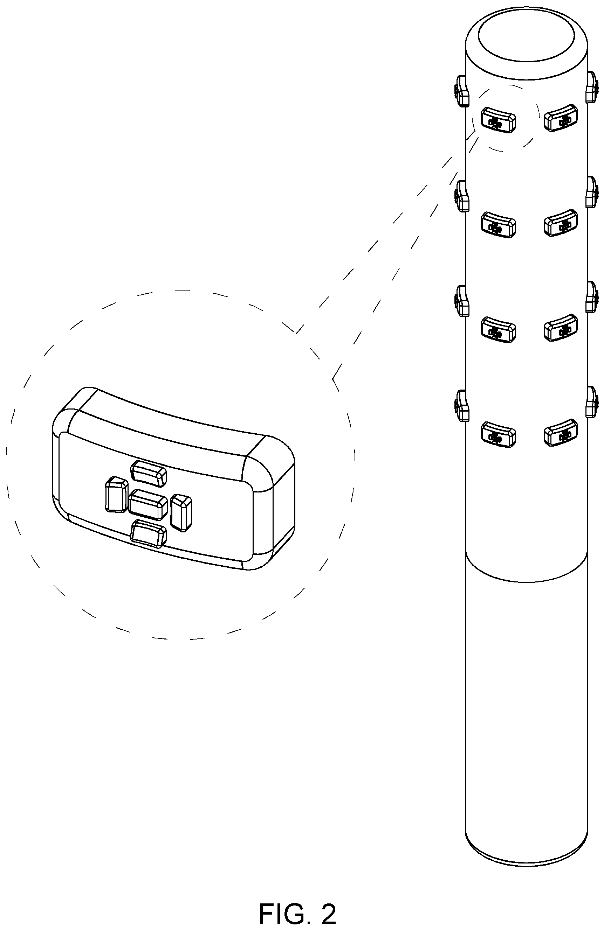

Figure 2: Shows the arrangement of the sensors in bands around the stick, detailing their orientation to capture comprehensive images and 3D video from multiple angles.

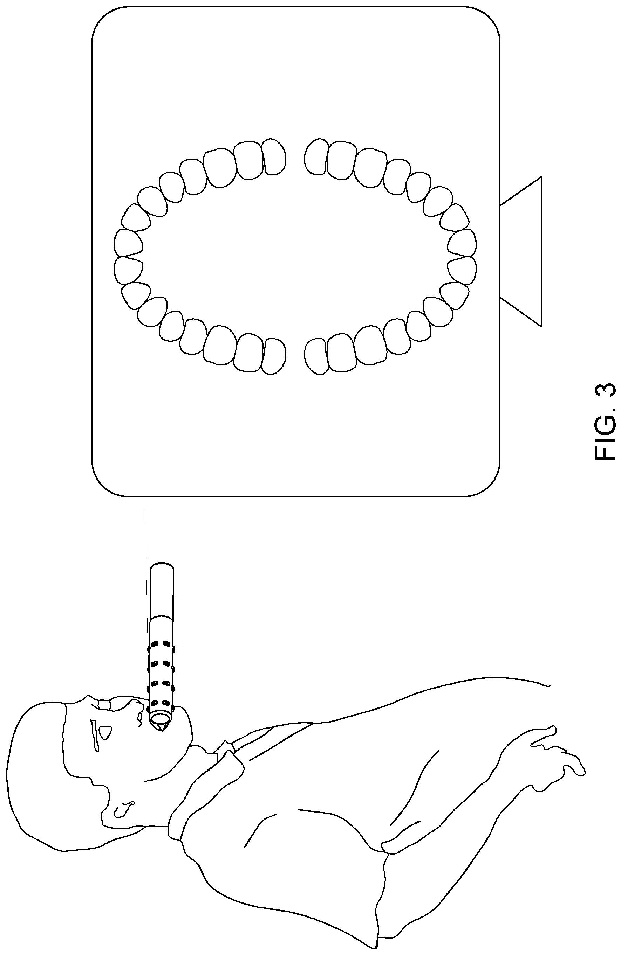

Figure 3: Illustrates the clinical procedure for capturing full arch scans with a single click, showing the orientation of the device relative to the upper and lower arches.

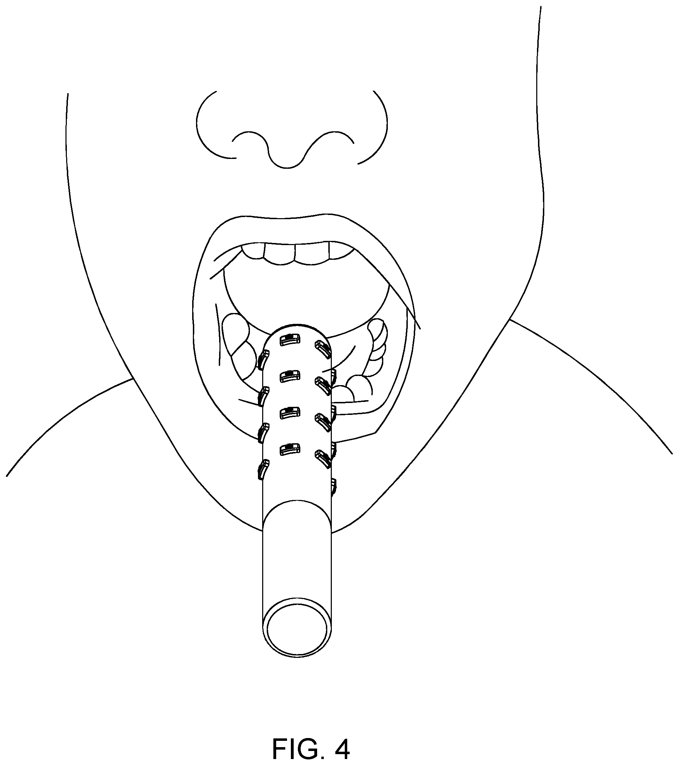

Figure 4: Explains the bite registration process, where the device is positioned against the cheek to capture the relationship between the upper and lower arches.

### Detailed Description of the Invention:

The invention comprises several key features and procedures:

### Clinical Workflow Integration:

The intraoral photogrammetry device is designed for seamless integration into dental practice workflows. The straightforward procedure reduces the need for multiple devices and steps, improving efficiency and accuracy in digital impression taking. The single-click capture system minimizes patient discomfort and chair time, making it an ideal tool for high-precision dental procedures, such as implant placement, orthodontics, and restorative dentistry. The device's instant processing capabilities allow for real-time analysis and adjustments, enhancing the overall quality of care.

By innovating in areas where existing technologies fall short, this intraoral photogrammetry device offers a significant advancement in the field of digital dentistry, providing practitioners with a more reliable, efficient, and user-friendly tool for capturing detailed oral data.

### 1. Device Configuration:

Stick-Like Design: The device is shaped like a stick or wand, designed to be easily maneuvered within the oral cavity.

### Camera Sensors:

Tip Sensor: Positioned at the end of the stick, facing directly up at a 90-degree angle relative to the stick.

Three/Four Bands of Sensors: Each band contains multiple micro cameras positioned around the circumference of the stick. These sensors are oriented at 45 degrees to the left, right, front, and back.

Multi-Functionality: Each sensor can capture high-resolution photo images and 3D video, enabling a detailed capture of the oral cavity from multiple angles simultaneously.

Known Structures Identification: The device is capable of recognizing known structures within the oral cavity that have been pre-marked with random surface textures, allowing for precise 3D spatial orientation.

### 2. Procedure:

### Single Click Full Arch Scanning:

Upper Arch: The device is inserted into the central cavity of the mouth, perpendicular to the occlusal plane. A single click initiates the capture, where all sensors simultaneously take a photo and 3D video, capturing the entire upper arch.

Lower Arch: The same process is repeated with the device oriented for the lower arch.

Data Processing: The captured data from each arch is instantly processed to generate a 3D STL file and a detailed color map, providing a full digital impression of both arches.

### Bite Registration:

Cheek Side Capture: The device is held against the inner cheek, with a single click capturing the relationship between the upper and lower arches on each side. This process captures the bite registration data accurately by using the 45-degree oriented sensors.

Instant Processing: The bite registration is immediately calculated and merged with the full arch scans in the 3D CAD software, ensuring the digital models are aligned correctly. Clinical Workflow Integration: The device's simple and fast capture process allows it to be seamlessly integrated into clinical workflows, reducing chair time and improving patient comfort.

### 3. Distinguishing Features from Existing Technologies:

Single Device Full Capture: Unlike existing systems that require separate devices or multiple steps to capture full arch, color, and bite registration, this device accomplishes all tasks with a single tool and minimal actions.

Instant Data Processing: The real-time processing of captured data to generate 3D models and color maps instantly distinguishes this device from current intraoral scanners and photogrammetry systems, which often require post-processing steps.

Advanced Sensor Orientation: The unique configuration of multiple camera bands at different angles allows for comprehensive coverage of the oral cavity in a way that single-line scanners and traditional photogrammetry systems cannot achieve.

Known Structure Recognition: The device's ability to identify and reference known structures within the mouth for precise spatial orientation during scanning is a novel feature not commonly found in existing devices.

## Claims

1. An intraoral photogrammetry device, comprising:
a stick-like body designed for insertion into the oral cavity;
a sensor positioned at the tip of the stick-like body;
at least three sensor bands positioned around a perimeter of the stick-like body, each sensor band comprising a plurality of sensors, the plurality of sensor bands being spaced along a length of the stick-like body;
wherein each sensor is capable of capturing both photo images and 3D video; and
means for capturing a full-arch scan, color map, and bite registration with a single click, using the data from all sensors simultaneously.

2. The device of claim 1, wherein the sensor configuration is designed to capture comprehensive 3D data and color maps for the entire oral cavity in a single action per arch.

3. The device of claim 1, further comprising a recognition system that identifies known structures within the oral cavity, marked with random surface textures, for precise spatial orientation and reference within the 3D model.

4. The device of claim 1, wherein the captured data is processed instantly and delivered to 3D CAD software to generate 3D STL files and color maps without the need for post-processing.

5. The device of claim 1, wherein the cheek side capture procedure accurately registers the bite relationship between the upper and lower arches, integrating this data into the full arch scans.

6. The device of claim 1, wherein the sensors are designed to function effectively in the challenging lighting conditions of the oral cavity, reducing the impact of shadows and reflections.

7. The device of claim 1, wherein the sensor bands are positioned to provide maximum coverage of the oral cavity, capturing detailed images even in complex anatomical areas such as the posterior regions of the mouth.

8. The device of claim 1, wherein the entire capture and processing sequence is designed to reduce clinical chair time and improve patient comfort during the scanning procedure.

9. The device of claim 1, wherein each sensor band comprises four sensors equi-spaced around the perimeter of the stick-like body.
